Europäisches Patentamt

European Patent Office

Office européen des brevets

(11) Publication number: **0 442 175 A1**

# EUROPEAN PATENT APPLICATION

(21) Application number: 90301493.4

(22) Date of filing: 13.02.90

(51) Int. Cl.⁵: **A01H 1/02, A01H 4/00**

(43) Date of publication of application:
21.08.91 Bulletin 91/34

(84) Designated Contracting States:
AT BE CH DE DK ES FR GB GR IT LI LU NL SE

(71) Applicant: **PIONEER HI-BRED
INTERNATIONAL, INC.**
**700 Capital Square 400 Locust Street
Des Moines Iowa 50309(US)**

(72) Inventor: **Tomes, Dwight T.
8 Beechwood
Cumming, Iowa 50061(US)**

(74) Representative: **Goldin, Douglas Michael et al
J.A. KEMP & CO. 14, South Square Gray's Inn
London WC1R 5EU(GB)**

(54) **Plants with increased tissue culture and regeneration potential.**

(57) This invention relates to plants with genetically increased tissue culture and regeneration potential. These plants provide seeds and plants therefrom which also display the genetically increased tissue culture and regeneration potential. These plants are produced by initiating embryogenic callus culture on a defined culture medium; maintaining the embryogenic callus culture for a specific length of time; selecting for those embryogenic callus that have organized into discrete, more advanced globular structure, a portion of which germinates to form a plant; and regenerating the plant produced therefrom which is capable of seed production. Thus, this invention is also directed to methods for selecting plants with an genetically increased tissue culture and regeneration potential. This invention is also directed to a genetic factor derived from plants, which is capable of conferring an genetically increased tissue culture and regeneration potential to plant and to the plant's progeny.

## PLANTS WITH INCREASED TISSUE CULTURE AND REGENERATION POTENTIAL

Field of Invention

This invention relates to plants with genetically increased tissue culture and regeneration potential. These plants provide seeds and plants therefrom which also have genetically increased tissue culture and regeneration potential. This invention is also directed to methods for selecting plants with increased tissue culture and regeneration potential.

## BACKGROUND OF THE INVENTION

Recombinant DNA technology has been a stimulus for studying tissue culture of plants. Tissue culture methods are recognized as potentially powerful tools to advance plant genetics. Once the structure and function of a plant gene is known, the next step is to insert the cloned gene into a plant cell in order to express the gene. After insertion of the cloned gene into the plant cell, the transformed plant cell is regenerated into a whole plant.

Plant regeneration makes possible recovery and incorporation of new and useful genes into seed which can be used in a breeding program. Starting a plant tissue culture requires a responsive cell from the plant, a suitable environment, a procedure to promote cell and tissue growth and, lastly, a series of culture environments to recover a plant from the culture. Another factor which dramatically influences the ability to establish cultures and regenerate plants is the plants' genetic potential to respond to the culture environment in an efficient and predictable fashion.

Current tissue culture methods permit some form of plant regeneration from cultures of all the major cereal crops. Despite this, the techniques for cellular manipulation of these species still lag seriously behind those of the model species such as tobacco and petunia. The potential benefit of tissue culture technology for studies on crop improvement depends on effective methods to grow somatic cells in vitro. However, the regeneration potential of callus and/or suspension cultures decreases with time (Orshinsky, B.R. and D.T. Tomes, J. Plant Physiol. 119:389-397 (1985)). The regeneration potential varies among genotypes, especially as length of the culture period increases. The isolation of genotypes which have both an increased ability to initiate callus and suspension cultures and to retain regeneration ability over relatively long periods (12 months or longer) are especially valuable. Callus and suspension cultures from such genotypes can be manipulated more easily and over the time periods required for in vitro selection, and genetic transformation experiments. Such cultures can also provide a source of totipotent protoplasts, which are very important for many applications of plant transformation with genetically engineered genes.

## SUMMARY OF THE INVENTION

This invention relates to plants with genetcally increased tissue culture and regeneration potential. These plants provide seeds and plants therefrom which also display the genetically increased tissue culture and regeneration potential. These plants are produced by 1) initiating embryogenic callus culture on a defined culture medium; 2) maintaining the embryogenic callus culture for a specific length of time; 3) selecting for those embryogenic calli that have organized into discrete, more advanced globular structures, a portion of which germinates to form a plant; 4) and regenerating the plant produced therefrom which is capable of seed production. Thus, this invention is also directed to methods for selecting plants with an increased tissue culture and regeneration potential. This invention is also directed to a genetic factor derived from plants, which is capable of conferring an increased tissue culture and regeneration potential to the plant and to the plant's progeny.

## DEFINITIONS

In the description and examples, that follow, a number of terms are used herein. In order to provide a clear and consistent understanding of the specification and claims, including the scope to be given such terms, the following definitions are provided:

Tissue Culture includes the culture of plant tissues, cells or protoplasts. Further, the cells, protoplasts or tissues which can be employed in the process of the invention can have originated from explants of haploid as well as diploid plants. Still further, haploid tissue cultures can be obtained directly from immature pollen contained within anthers. Accordingly, the term "culturing tissue" and similar terms encompass the propagation of tissue, cells or protoplasts by the herein described "tissue culture" process.

Subculture is defined as transferring or moving callus or suspension cells from old culture medium to fresh culture medium, usually under conditions which maintain the (axenic) sterility of the culture.

Callus (embryogenic callus) is defined as either a compact or soft (friable) tissue with only very early stages of organization (embryoids) on maintenance medium but which on regeneration medium organizes into discrete, more advanced globular structures, a portion of which germinates to form plants.

Friable means easily crumbled or pulverized, and are callus or suspension cultures which grow rapidly as soft or finely dispersed cell suspensions in liquid medium.

Regenerated, referring to plants, means plants derived from cell and tissue culture.

Progeny, means plants obtained by self-fertilization, sibling-fertilization, back-crossing or out-crossing of the regenerated plants.

## DETAILED DESCRIPTION OF THE INVENTION

This invention relates to plants that have an increased regeneration potential. Plants that have the increased regeneration potential include both monocotyledons and dicotyledons.

All plants from which protoplasts can be isolated and cultured to give whole regenerated plants can be transformed into plants which have increased regeneration potential. In addition, there is an increasing body of evidence that practically all plants can be regenerated from cultured cells or tissues, including, but not limited to, all major cereal crop species, sugarcane, sugar beet, cotton, fruit and other trees, legumes and vegetables. Some plants include, for example, species from the genera Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum, Sorghum, Datura, Ipomoea, Passiflora, Cyclamen, Malus, Prunus, Rosa, Rubus, Populus, Santalum, Allium, Lilium, Narcissus, Annanas, Arachis, Phaseolus, Pisum, Oryza, Avena, Dancus, and Saccharum.

Parts obtained from the regenerated plant, such as flowers, seeds, leaves, branches, fruit, and the like are covered by the invention provided that these parts comprise cells with increased regeneration potential. Progeny, variants, and mutants of the regenerated plants are also included within the scope of this invention.

As used herein, increased regeneration potential means that the plants are more responsive to initiation of callus cultures than their respective parental line. According to this invention, the increased regeneration potential can be transmitted genetically to the plants' progeny by conventional breeding techniques or by use of tissue culture techniques. Importantly then, the plants of this invention with increased regeneration potential can be used in crosses to confer regeneration potential. These plants can be sexually reproduced to provide seeds and plants therefrom which display inherited regeneration potential.

This invention arose from the development of a mutated corn line in cell culture. This cell line exhibited reproducible, significantly increased tissue culture and regeneration potential. Prior to this invention, corn was known to have poor regeneration potential. It was not known prior to this invention that there is a gene or "genetic factor" that determines increased tissue culture and regeneration potential in plants. Moreover, the inventors have determined that this gene or genetic factor is capable of being transmitted by inheritance to the progeny of the plants.

This invention is therefore also directed to methods for fostering or establishing this increased regeneration potential in existing plant cell lines. This invention is further directed to novel plant lines having increased regeneration potential that are produced according to the screening methods of this invention.

One method according to this invention comprises the selective subculture of callus on a defined culture medium for a time sufficient to isolate callus with increased tissue culture and regeneration potential. The callus is selectively cultured such that only those areas which retain the embryogenic phenotype with early stage embryoids are maintained. A culture period in excess of three months, preferably six months and more preferably ten to twelve months is required for expression of genetically increased culture initiation and regeneration potential.

By the term "defined-culture medium" is meant a medium which will support the growth of the plant tissue cell being cultured. The defined culture medium employed in the method of this invention can therefore comprise any tissue culture medium capable of supporting tissue growth. Those skilled in the art are aware of the particular requirements to culture tissues of a particular plant species and to subsequently cause differentiating growth to form a plant therefrom.

3

The present invention utilizes tissue culture technology to isolate, characterize and develop plant cell lines, which have a higher regeneration potential. The basic procedural manipulative techniques, including culture materials and procedures are considered within the skill of the art and thus the procedures and materials are not discussed or described in detail. Those skilled in the art are aware of the particular requirements to culture tissues of a particular plant species and to subsequently cause differentiating growth to form a plant therefrom. For a general discussion of tissue culture techniques, attention is directed to "Plant Tissue and Cell Culture," H.E. Street, ed., University of California Press (in two editions 1973, 1977).

The method of this invention will be described in detail concerning maize, or corn, but is not intended to be limited thereto. In fact, since corn is know to have poor culture and regeneration potential, this invention can readily be extended to cell lines of other plant species, although the results may not be as dramatic by comparison with prior art results.

Maize embryogenic callus cultures are characterized into two types. Type I culture is a firm, compact, translucent, convoluted proliferation, with relatively slow growing callus. Type II culture is a soft, highly friable, rapidly growing callus with well-formed somatic embryos. Type I embryogenic callus cultures are useful for several applications such as short-term regeneration for observing somaclonal variation and for other uses where the time in culture is minimized. Because of their rapid growth rate, stability and ease of regenerating plants over a prolonged period, Type II cultures excel for in vitro selection and transformation studies with protoplasts.

The method for producing plants with increased regeneration potential comprises the following steps:

1) growing plants to maturity under vigorous growth conditions either in a greenhouse or in the field;

2) crossing plants of an inbred line by self or sibmating and allowing immature embryos to attain 1-2 mm size;

3) removing embryos from kernels and placing embryos axis down on a defined culture medium;

4) isolating Type II callus after culturing for about 14 days at 27° C in the dark and continuing to subculture the callus according to the screening method of this invention. Preferably the callus is subcultured at 7 day intervals on a defined culture medium containing 2,4-D for a period of time greater than 3 months, preferably greater than 6 months, and more preferably for 10 months or longer;

5) regenerating plants by medium sequence which removes auxin and allows somatic embryos to germinate; and

6) growing plants in a growth chamber, or a green house, to allow flowering and seed set.

The method described above is targeted to regenerating corn. However, the method of this invention is not intended to be limited to corn. Plants which may be both produced and screened for this increased regeneration potential include all dicotyledons and monocotyledons. The invention is particularly directed to the cereal crops such as rye, barley, wheat, sorghum, oats, millets, rice, sunflower, and soybean. Thus, for example, similar methods can be adapted for sunflower by placing a responsive tissue, including seedling hypocotyls in 2mm segments onto a defined culture medium.

In one embodiment of this invention, novel inbred corn lines possessing superior tissue culture response, i.e. regeneration potential, were developed from corn inbred B73. Inbred B73 is a commercially desirable parent of hybrid corn which has a very low culture response which inhibits its ability to be improved using cell and molecular biology, for such procedures as in vitro selection and genetic transformation. Corn inbreds designated as T66, W62, R21 are from 10 to 100 fold more responsive to initiation of callus cultures than their parent line, inbred B73. These inbred lines were obtained by recovering plants capable of seed production from a long-term callus culture (older than 12 months) of inbred B73. Seed-derived from regenerated plants of a specific B73 callus culture retain 10-100 fold increase in initial and subsequent Type II culture response compared to other tissue culture and seed derived plants of inbred B73. The resultant seed of T66, W62, and R21 are similar to inbred B73 for many agronomic attributes, are as high to slightly lower per se yield, equal in test cross performance while still retaining superior in vitro culture response, namely 10-100 fold increased culture initiation responsiveness for Type II culture response.

In another embodiment of the invention, corn inbreds designations K29 and G50 were tissue cultured in accordance with the invention. Selfed seed from the plants which are regenerated from these cultures show two-fold increase in their ability to form Type I cultures (G50) or a two-fold increase in total (Type I and II) culture response (K29). These tissue culture- derived inbreds have electrophoresis patterns which are expected for the respective inbreds.

In yet another embodiment of the invention, hypocotyls from sunflower germplasm accession SMF3 are cultured according to the method of Patterson et al., Plant Science 42:125 (1985). After a period of 3 months, plants are regenerated and grown to maturity, and selfed in the greenhouse. When these $R_1$

seedling hypocotyls are again recultured, the proportion of segments which regenerate plants is increased from 50% to 100%. In addition the proportion of segments which produced regenerated plants also increased to nearly 100%. $R_1$ is the first self-pollinated seed generation from a tissue culture derived plant. The regenerated plant is $R_0$, $R_2$ is seed from $R_1$ plants, etc.

The novel plants and the plants produced according to the methods of this invention can be used in various ways. The plants can be used in tissue culture techniques to improve the plant using in vitro selection. Also, according to this invention, plant cells with increased regeneration potential can be transformed with a gene using genetic engineering techniques. Using either in vitro selection or genetic transformation, the selected or transformed plant can be crossed with a second parental line to produce hybrid plants.

The following examples are presented for purposes of clarity and understanding, and are not intending to be limiting.

**EXAMPLE 1**

**DATA ON INBRED CORN LINES T66, W62, AND R21**

The following example provides data on inbred corn lines T66, W62, and R21. These inbred lines were obtained by recovering plants capable of seed production from a long-term callus culture (older than 12 months) of inbred B73.

## Table 1

### Tissue Culture Response--Percent Embryos Giving Rise to Type I and Type II Embryogenic Callus from TC2 Plants Regenerated from Inbred B73

| Pedigree Identity | N | 28-Day Response | |
| --- | --- | --- | --- |
| | | % Type I | % Type II |
| 694#3(*13 | 27 | 0 | 29.6 |
| 15 | 54 | 0 | 18.5 |
| 11A | 37 | 0 | 40.5 |
| 710#1)**11 | 42 | 0 | 21.4 |
| | 50 | 2.0 | 28.0 |
| B73 | 75 | 0 | 0 |
| | 170 | 0 | 0 |

*Progenitor of T66.

**Progenitor of R21.

## Table 2

Percent Embryos Giving Rise to Type I and Type II
Embryogenic Callus after 14 and 28 Days in Culture from
Seed Derived B73 and Several Tissue Culture
Derived Plants from Inbred B73

| Pedigree | N | 14-Day Response | | 28-Day Response | | |
|---|---|---|---|---|---|---|
| | | Type I | Type II | Type I | Type II | |
| B73 694)T3X[1] | 471 | 0.6 | 8.3 | 0.6 | 3.1 | C |
| B73-TC694#11B73-TC694#2)TX[2] | 380 | 2.0 | 8.0 | 0.3 | 8.9 | B |
| B73-TC710#1-/B73-TC 694#3-)X[3] | 711 | 1.0 | 31.4 | 1.8 | 25.7 | A |
| Other B73 regenerates[4] | 2,080 | 0.1 | .5 | 0.0 | .2 | D |
| B73[5] | 279 | 0.0 | .1 | 0.0 | .6 | D |

[1]Progenitor of T66.
[2]Progenitor of W62.
[3]Progenitor of R21.
[4]Summary from 15 regenerated plants.
[5]Plants from seed derived B73.

## Table 3

Percent Embryos Giving Rise to Type I and Type II
Embryogenic Callus after 14 and 28 Days in Culture from
Inbreds T66, W62, and R21 Derived from B73 Callus and
from Seed Derived from B73

| Pedigrees | N | 14-Day Response | | 28-Day Response | |
|---|---|---|---|---|---|
| | | Type I | Type II | Type I | Type II |
| T66* | | | | | |
| B73-TC694-1-)T371 | 150 | 0.0 B | 32.7 CD | 0.0 | 30.0 |
| W62* | | | | | |
| B73-TC694#1-/B73- | | | | | |
| TC694-)17X | 60 | 0.0 B | 58.3 BC | 0.0 | 45.0 |
| R21* | | | | | |
| B73-TC710#1-/B73- | | | | | |
| TC694#5)2X | 80 | 3.8 B | 3.8 D | 0.0 | 6.3 |
| B73 | 277 | 0.0 B | 0.0 B | 0.0 | 0.0 |

*Date shown from same ears used for per se and top cross
yield data.

## TABLE 4

Agronomic characteristics of T66, W62, and R21 and
B73 per se and with two top cross testers, G50 and
HD22 from three locations

| | BU ACR | Grain MST | GDU SHD | STK LDG | % Not RT LDG | STA GRN | TST WT | GRN Qual | SDG VGR | EST CNT | Inches PLT HT | EAR HT |
|---|---|---|---|---|---|---|---|---|---|---|---|---|
| T66/G50 | 152 | 19.3 | 1275 | 92 | 94 | 3.3 | 61 | 8.4 | 4.8 | 59 | 121 | 56 |
| /G35 | 165 | 19.3 | 1378 | 92 | 82 | 4.2 | 58 | 7.7 | 4.9 | 51 | 124 | 57 |
| W62/G50 | 148 | 19.1 | 1300 | 94 | 90 | 3.0 | 60 | 7.7 | 4.2 | 56 | 120 | 58 |
| /G35 | 168 | 20.1 | 1383 | 91 | 72 | 4.2 | 59 | 7.1 | 4.6 | 57 | 124 | 56 |
| B73/G50 | 163 | 18.9 | 1290 | 95 | 88 | 4.0 | 60 | 7.8 | 4.6 | 56 | 121 | 56 |
| /G35 | 170 | 19.9 | 1370 | 93 | 76 | 5.0 | 58 | 7.5 | 4.9 | 57 | 125 | 55 |
| R21/G50 | 145 | 18.7 | 1290 | 92 | 87 | 2.7 | 60 | 7.7 | 4.3 | 55 | 120 | 58 |
| G35 | 160 | 19.4 | 1365 | 93 | 72 | 3.6 | 58 | 7.5 | 5.2 | 57 | 123 | 55 |
| R21 | 45 | 19.5 | 1480 | 99 | -- | 2.3 | 59 | 8.5 | 4.6 | 40 | 89 | 38 |
| T66 | 46 | 20.1 | 1525 | 92 | -- | 3.0 | 59 | 7.6 | 4.5 | 42 | 92 | 44 |
| W62 | 62 | 20.6 | 1450 | 97 | -- | 3.2 | 61 | 8.1 | 6.5 | 48 | 94 | 42 |
| B73 | 84 | 20.1 | 1980 | 97 | -- | 4.3 | 60 | 8.5 | 5.3 | 52 | 98 | 45 |

GRAIN MST = % moist at harvest

GDU SHD = heat units to 50% shed

STK LDG = % not stalk lodged

RT LDG = % not root lodged

STAG RN = stay green score (1-9) 1 = poor

TST WT = test weight lbs/bu at 15.5% mst

GRN QUAL = grain quality score (1-9) 1 = poor

SDG VGR = seedling vigor score (1-9) 1 = poor

EST CNT = early stand count - no. of plants

PLT HT = plant height - inches

EAR HT = ear height - inches

EP 0 442 175 A1

EP 0 442 175 A1

## TABLE 5

Comparison of morphological attributes of T66, W62,
R21 and B73 grown at Johnston, Iowa

| Genotype | Pollina-tion | Length Central Spike | # Primary Tassel Branch | # Secondary Tassel Branches | Width-Ear Leaf | Length Ear Leaf | Ear Height (cm) | Plant Height (cm) |
|---|---|---|---|---|---|---|---|---|
| B73 | 88.8±2.1 | 4.2±0.4 | 7.9±2.6 | 1.5±0.6 | 10.5±0.8 | 81.6±3.6 | 104.5±9.4 | 232.5±8.6 |
| T66 | 85.2±2.3 | 4.3±0.4 | 8.8±1.5 | 1.8±0.4 | 10.8±0.6 | 78.2±3.8 | 111.0±7.4 | 234.4±3.8 |
| W62 | 81.8±1.8 | 4.2±0.4 | 12.4±1.7 | 2.0±0.0 | 10.4±1.0 | 75.2±4.6 | 98.0±2.7 | 221.2±5.8 |
| R21 | 86.2±2.3 | 4.4±0.4 | 7.2±1.9 | 2.0±1.9 | 9.5±0.8 | 72.1±2.9 | 87.2±14.1 | 210.4±12.7 |

Each value is the mean of 5 plants

## Table 6

Plant Regeneration from T66, W62, R21, and B73
Embryogenic Cultures Approximately
28 Days after Culture Initiation

| Genotype | Number Embryos | $\underline{X}$ Plants/Embryo |
|---|---|---|
| T66 | 67 | 2.10 |
| W62 | 84 | 1.90 |
| R21 | 14 | 2.85 |
| B73 | 2 | 2.50 |
| B73 Regenerate | 1 | 1.00 |

## Table 7
### Electrophoresis Data

| Loci | B73 | T66 | W62 | R21 |
|------|-----|-----|-----|-----|
| Aco1 | 4 | 4 | 4 | 4 |
| Acp1 | 2 | 2 | 2 | 2 |
| Adh1 | 4 | 4 | 4 | 4 |
| Adk1 | 4 | 4 | 4 | 4 |
| Amp1 | 4 | 4 | 4 | 4 |
| Amp3 | 5 | 5 | 5 | 5 |
| Cat3 | 9 | 9 | 9 | 9 |
| Dia1 | 8 | 8 | 8 | 8 |
| Dia2 | 4 | 4 | 4 | 4 |
| Enp1 | 6 | 6 | 6 | 6 |
| E8 | 5 | 5 | 4.5 | 5 |
| Glu1 | 7 | Null[2] | 7 | Null[2] |
| Got1 | 4 | 4 | 4 | 4 |
| Got2 | 4 | 4 | 4 | 4 |
| Got3 | 4 | 4 | 4 | 4 |
| Gpt | 4 | 4 | 4 | 4 |
| GS | 4 | 4 | 4 | 4 |
| Hk2 | 2 | 2 | 2 | 2 |
| Idh1 | 4 | 4 | 4 | 4 |
| Idh2 | 4 | 4 | 4 | 4 |
| Mdh1 | 6 | 6 | 6 | 6 |
| Mdh2 | 3.5 | 3.5 | 3.5 | 3.5 |
| Mdh3 | 16 | 16 | 16 | 16 |
| Mdh4 | 12 | 12 | 12 | 12 |
| Mdh5 | 12 | 12 | 12 | 12 |
| MMM | M | M | M | M |
| Pgd1 | 3.8 | 3.8 | 3.8 | 3.8 |
| Pgd2 | 5 | 5 | 5 | 5 |
| Pgm1 | 9 | 9 | 9 | 9 |
| Pgm2 | 4 | 4 | 4 | 4 |
| Phi1 | 4 | 4 | 4 | 4 |

### Table 7
#### Electrophoresis Data

| Loci | B73 | T66 | W62 | R21 |
|------|-----|-----|-----|-----|
| TPI1 | 4 | 4 | 4 | 4 |
| TPI2 | 4 | 4 | 4 | 4 |
| TPI3 | 4 | 4 | 4 | 4 |
| TPI4 | 4 | 4 | 4 | 4 |

[1]Allele numbers are laboratory designations of specific alleles.

[2]These samples were found with leaf tissue which can often lead to loss of Glu1 activity; thus, these readings may not be true nulls.

## EXAMPLE 2

The following example provides data on inbred corn lines G50 and K29. The tissue culture derived versions of these inbreds were obtained by recovering plants capable of seed production from long-term callus cultures (older than 10 months) of the respective inbreds G50 and K29.

Table 8


Percent Embryos Giving Rise to Type I and Type II
Embryogenic Callus after 14 and 28 Days in Culture from
Seed Derived G50 and Tissue Culture Derived G50
and for Seed Derived and Tissue Culture

1985

|  |  | 14-Day Response |  | 28-Day Response |  |
|---|---|---|---|---|---|
| Pedigree | N | Type I | Type II | Type I | Type II |
| G50 (TC-3 regen)[1] | 470 | 37.1 | 0.8 | 27.6 | 0.0 |
| G50 (Seed)[2] | 190 | 24.6 | 0.5 | 22.4 | 0.0 |

1987
14-Day Response

| Pedigree | N | Type I | Type II |
|---|---|---|---|
| G50-(TC-4833)1[3] | 120 | 22.3 | 0.00 |
| G50-(Seed)[2] | 50 | 8.0 | 0.00 |
| K29-(TC4246)1[4] | 140 | 24.4 | 25.8 |
| K29-(Seed)[2] | 80 | 19.0 | 0.00 |

[1] Summary from 3 regenerated plants, callus < 3 months old.
[2] Plants from seed lot of inbreds.
[3] Summary callus > 10 months old.
[4] Summary of one regenerated plant, callus > 10 months old.

**EXAMPLE 3**

The following example provides data on the seed line SMF3 of sunflower. The lines designated PT001, PT003, PT016, PT019, PT021, and PT024 were derived through a cycle of callus initiation and plant regeneration followed by cross-pollination of the resultant plants in the greenhouse.

Data is also provided on the seed lines PT 021-R1 and PT 024-R1 which were derived from tissue culture selection for regeneration from the sunflower germplasm SFM3 on HAR culture medium (K.E. Patterson and N.P. Everett, Plant Science 42:125-132 (1985).

Number of seedlings and explants/seedlings which gave rise to regenerated plants following culture on

14

HAR medium from SFM3 germplasm and its R1 descendants.

### TABLE 9

| Genotype | Seedlings | | | Explants | | |
|---|---|---|---|---|---|---|
| | N | # regenerated | % | N | regenerating | % |
| SMF3[1] | 155 | 80 | 52 | -- | -- | -- |
| R1-PT001[2] PT003 | 4 | 3 | 75 | -- | -- | -- |
| R1-PT016[2] | 3 | 2 | 67 | -- | -- | -- |
| PT019 | 7 | 5 | 71 | 76 | 24 | 32 |
| R1-PT021[2] | 8 | 8 | 100 | 73 | 52 | 71 |
| R1-PT024[2] | 34 | 34 | 100 | 204 | 196 | 96 |

[1] SMF3 USDA germplasma release 3/18/83

[2] Progeny of single regenerated plants (Ro) of SMF3

## EXAMPLE 4

### ESTABLISHING CALLUS CULTURES FROM CORN

#### Growing Plants for Establishing Callus Cultures

Plants should be grown under environmental conditions which produce abundant pollen, a minimum of silk delay, and which give excellent seed set upon pollination. Once the inbred or hybrid of choice has been pollinated by either self, sib, or cross pollination, the ear shoot is covered to prevent stray pollen from contaminating the initial pollination, and also to assure that embryo development will be relatively synchronized on the ear.

#### Checking for Proper Embryo Size to Start Callus Cultures

Zygotic embryos which are between 1-2 mm in length are the most efficient for callus initiation. Embryo development is monitored while the ear is still on the corn plant so that the best size embryo can be used. The husks on the ear are split and moved back so that the middle portion of the ear is exposed. The embryo is always located on the side of the kernel that is opposite the point of ear attachment to the stalk. The upper one-third of a kernel is removed with a scalpel and the immature embryo located with a microspatula which removes the developing endosperm from the dissected kernel. The embryo is often removed with the endosperm. Either a scalpel which has a metric scale on the handle or a separate rule can be used to determine the embryo length (long axis of the embryo). If the embryo is of the proper size, either a half or full ear can be removed for culture. If the embryos as too small, the ear should be left for another day or two. An embryo of 0.5 mm will normally develop to a useful size of 1-2 mm in 24 hours under summer field conditions in Iowa. If the embryos are above 2.5 mm, the best advice is to throw away the ear and look for others that were pollinated more recently.

#### Preparing Ears for Culture

Ears with the proper size embryo can be prepared for culture as follows. If one-half the ear will be used for culture and the other half used as a control for genetic studies, use a pruning shear to cut the ear at the midpoint of pollinated kernels. The husk should be left around the ear to protect kernels on either side of the cutting mechanism. The top half can be used for culture. The portion of the ear remaining on the plant should be treated with a fungicide such as CAPTAN™ on the cut end to prevent ear deterioration and the remaining husk returned to its natural position. A pollinating bag can be placed back over the ear to protect from undue desiccation. The half ear for culture can be used immediately or stored for up to 48 hours for later culture. If the ear will be stored before culture, the ear should be placed in a moisture-proof, plastic bag and stored at 4° C until culturing can be done.

Within the ear, embryos are sterile, but contamination (fungal spores, bacteria, etc.) may be introduced by sampling for embryos and by harvesting ears for tissue culture. Fungal spores, bacteria, etc., are removed or killed by a surface sterilization treatment with household bleach (hypochlorite solution) and water. All the husks and remaining silks should be removed and the ear trimmed on both ends. If the ear is too large to fit in a 100 mm x 80 mm storage jar, it should be cut into two pieces. Dehusking should be done in a location separate from that used for culturing since the husks are primary source of fungal spores, bacteria, and also insects which can later contaminate a culture room.

Once the ear is in the storage jar, a solution consisting of 50% household bleach with the addition of 2-3 drops of Tween-20 (a surfactant) per 500 mls solutions is used for surface sterilization. Once the ear is placed in the bleach solution, the covered storage jar is left in mild vacuum (from a sink aspirator) for 10 minutes. The ear should also be periodically agitated to make sure that all surfaces are sterilized. Every operation which follows must be done under aseptic or sterile conditions. Following surface sterilization, the vacuum is removed and the bleach solution aseptically poured from the container. In a protected (sterile) environment, sterile deionized water is poured into the container with the ear and soaked for 1-2 minutes. After 1-2 minutes of soaking, the ear can be transferred aseptically to another storage jar containing sterile deionized water.

## Starting the Callus Culture

Forceps are inserted into the small end of the ear to remove it from the sterile water and placed in a sterile petri dish. A scalpel is used to cut off the upper one-third of individual kernels in two kernel rows in the ear. Embryos are removed with a microspatula and placed with the embryo axis down on callus initiation medium (10 embryos/plate). The embryo axis side of the embryo is flat while the scutellum side is slightly curved. The process of dissecting and placing the embryos into culture medium should be done carefully because damaged embryos die instead of forming the desired type of callus. The culture medium for initiating callus consists of a mineral salt base including major and micronutrients and vitamins (CT base) with two important additions. The first is 2,4-D at 0.75 mg/1 and the two specific amino acids, proline at 1400 mg/1 and asparagine at 900 mg/1. Table 10 shows the culture initiation medium.

Once the embryos are placed on culture medium, the petri plates are placed in the dark in a controlled temperature incubator at 27° C.

## Table 10
### Culture Initiation Medium

| Macronutrients | mM | mg/1 |
|---|---|---|
| $KNO_3$ | 28.0 | 2,800 |
| $CaCl_2 \cdot 2H_2O$ | 2.0 | 300 |
| $MgSO_4 \cdot 7H_2O$ | 1.0 | 250 |
| $KH_2PO_4$ | 2.2 | 300 |
| $(NH_4)_2SO_4$ | 4.0 | 530 |
| $FeSO_4 . {}^7H_2O$ | 0.11 | 37.3 |
| $Na_2 . EDTA$ | 0.10 | 27.8 |
| | | |
| **Micronutrients** | | |
| $KI$ | 0.00045 | 0.75 |
| $H_3BO_3$ | 0.049 | 3.0 |
| $MnSO_4 \cdot H_2O$ | 0.060 | 10.0 |
| $2NSO_4 \cdot 7H_2O$ | 0.007 | 2.0 |
| $Na_2MoO_4 \cdot H_2O$ | 0.001 | 0.25 |
| $CuSO_4 \cdot 5H_2O$ | 0.0001 | 0.025 |
| $CuCL_2 \cdot 6H_2O$ | 0.0001 | 0.025 |
| | | |
| **Vitamins** | | |
| Nicotinic acid | 0.004 | 0.5 |
| Pyridoxine HCl | 0.002 | 0.5 |
| Thiamine HCl | 0.003 | 1.0 |
| Glycine | 0.027 | 2.0 |
| Myo-inositol | | 1,000 |
| | | |
| **Amino Acids** | | |
| Proline | 12 | 1,400 |
| Asparagine | 6 | 900 |
| Sucrose | | 20,000 |
| Gelrite | | 3,000 |
| 2,4-D | .0038 | 0.75 |

Observing Culture Quality

Many inbreds produce some Type I or Type II callus although few can be maintained for longer than 2-3 months before plant regeneration is reduced. The number of embryos which respond in the first 14 days and the subsequent ability of the callus to regenerate plants is genotype dependent. For example, B73 typically produces about four Type II calli per 1,000 cultured embryos. About 50% of these give rise to a stable callus line which can regenerate plans for one year or more. Hybrids with B73 have excellent initial response and also form callus lines which can regenerate plants over extended periods. Responsive

17

genotypes have been derived from plants regenerated from a long-term B73 callus culture. This genotype produced Type II callus from 15-30 embryos per 100 embryos cultured with excellent ability to form longer-term cultures with good plant regeneration.

The callus types which are observed and classified after 14 days are the following:

0 = no growth

1 = Type I callus consisting of compact, translucent, convoluted proliferation (shoot meristems produced by organogenesis) which, when touched with a scalpel, is relatively hard and organized.

2 = Type II callus consisting of a soft, rapidly growing callus with somatic embryos (round structures often sitting on a suspensor). These cultures are soft to the touch with a scalpel and, once established, are very rapid growing and can be used to initiate suspension cultures.

3 = Fluffy callus which has no somatic embryos or convoluted structures.

4 = Callus with translucent, leaflike structures. Type IV callus occasionally gives rise to a plant provided it is immediately transferred to a plant regeneration culture medium.

Type O and Type 3 callus are immediately discarded because they are not capable of later forming a plant. Both Type I and Type II callus can be grown for several subcultures and later regenerate plants and, thus, be used for in vitro selection and/or transformation.

Culture Maintenance

Type I and Type II calli produced after 14 days are dissected away from the remainder of the embryo and aseptically placed on fresh culture medium identical to that originally used for establishing cultures. The cultures are incubated in darkness at 27°C. At the end of a subsequent 14-day subculture, the Type I and Type II cultures are examined again. Type I cultures grow somewhat slower, with about half continuing to produce compact, translucent to light yellow calli with some shoots produced on this culture medium. Type II cultures consist of a soft base with somatic embryos continually formed on the surface. Type I cultures may revert to a Type I and Type II culture phenotypes to always examine callus for the presence of somatic embryos. If somatic embryos are not observed, the callus should be discarded because it will ultimately cease growth or else will not regenerate plants.

The present invention is not to be limited in scope by the cell line or seeds deposited, since the deposited embodiments are intended as single illustrations of one aspect of the invention and any cell lines or seeds which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims.

## Claims

1. A seed having genetically increased tissue culture response and regeneration potential and capable of germinating into a plant having genetically increased tissue culture response and regeneration potential and capable of producing seed.

2. A plant having genetically increased tissue culture response and regeneration potential and capable of producing seed wherein said seed is capable of germinating into a plant having genetically increased tissue culture response and regeneration potential and capable of producing seed.

3. The plant of Claim 2, wherein tissue from said plant is capable of germinating into a plant using tissue culture techniques and wherein said germinated plant has genetically increased regeneration potential.

4. A plant and the seed thereof regenerated from tissue culture wherein said plant has a genetically increased tissue culture response and regeneration potential.

5. The seed or plant of claims 1 to 4 wherein said seed or plant is a monocot or a dicot.

6. The seed or plant of claim 5 wherein said seed or plant is a monocot or dicot selected from the group of cereal and oil seed crops consisting of corn, rye, barley, wheat, sorghum, oats, millets, rice, sunflower, and soybean.

7. The seed or plant of claims 1 to 4 wherein said seed or plant is maize.

8. Inbred corn line according to Claim 1 having the designation T66.

9. Inbred corn line according to claim 1 having the designation W62.

10. Inbred corn line according to claim 1 having the designation R21.

11. Inbred corn line according to claim 1 having the designation K29.

12. Inbred corn line according to claim 1 having the designation G50.

13. Hybrid seed produced using the inbred seeds of one of claims 8-12 as at least one parental seed.

14. Inbred sunflower line according to claim 1 having the designation PT001, PT003, PT016, PT019, PT021, PT024.

15. A method for generating genetically increased tissue culture response and regeneration potential in a plant cell line, comprising the steps of:
    (a) initiation of embryogenic callus culture on a defined culture medium;
    (b) maintaining the embryogenic callus culture for a specific length of time; and
    (c) selecting for those embryogenic callus that have organized into discrete, more advanced globular structure, a portion of which germinates to form a plant; and
    (d) regeneration of a plant capable of seed production.

16. A plant produced by the method of claim 14.

17. A method of producing seeds which will produce plants with genetically increased tissue culture and regeneration potential, comprising:
    (a) growing a pair of parent plants wherein at least one parent carries a gene for increased tissue culture and regeneration potential;
    (b) cross-pollinating said parent plants to produce hybrid seeds F1; and
    (c) harvesting said hybrid seeds.

18. The method of claim 18 wherein said parent plants are monocots or dicots.

19. The method of claim 17, wherein said parent plants are monocots or dicots selected from the group of cereal and oilseed crops consisting of corn, rye, barley, wheat, sorghum, oats, millets, rice, sunflower, and soybean.

20. A genetic factor derived from plants, which genetic factor is capable of conferring an increased tissue culture and regeneration response to a plant and capable of transmission to said plant's progeny.

European. Patent Office

**PARTIAL EUROPEAN SEARCH REPORT**
which under Rule 45 of the European Patent Convention
shall be considered, for the purposes of subsequent
proceedings, as the European search report

Application number

EP 90 30 1493

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | JOURNAL OF PLANT PHYSIOLOGY, vol. 119, 1985, pages 389-397; B.R. ORCHINSKY & D.T. TOMES: "Effect of long-term culture and low temperature incubation on plant regeneration from a callus line of birdsfoot trefoil (Lotus coniculatus L.) | | A 01 H 1/02 A 01 H 4/00 |
| | * The whole article * | 1-6 | |
| A | | 15-20 | |
| | -- | | |
| X | DE-A-3 126 001 (NIPPON PAINT CO.) | | |
| | * Page 4, line 26 - page 11, line 10; claims 1,6 * | 1-5, 15-18 | |
| A | | 6,19, 20 | TECHNICAL FIELDS SEARCHED (Int. Cl.4) |
| | -- ./. | | A 01 H |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6,15-20

Claims searched incompletely:

Claims not searched: 7-14

Reason for the limitation of the search:

Claims 7-14: Art. 53b. of the European Patent Convention.

| Place of search | Date of completion of the search | Examiner |
|---|---|---|
| THE HAGUE | 30-10-1990 | DISSEN |

EPO Form 1505.1 03.82

| Category | Citation of document with indication, where appropriate, of relevant passages | Relevant to claim |
|---|---|---|
| | **DOCUMENTS CONSIDERED TO BE RELEVANT** | |
| A | N.W. SIMMONDS: "Principles of crop improvement", 1979, Longman, London, Chapter 2 "Basic features of plantbreeding", pages 27-39<br><br>* The whole article * | 1-6, 15-20 |
| | -- | |
| A | WO-A-88 10 066 (HARDY RESEARCH LABORATORIES INC.)<br><br>* Page 3, line 17 - page 8, line 29; claims 1-3 * | 1-6, 15 |
| | ---- | |

**CLASSIFICATION OF THE APPLICATION (Int. Cl. 4)**

**TECHNICAL FIELDS SEARCHED (Int. Cl. 4)**